# FASCICULE DE BREVET EUROPEEN

(11) **EP 3 385 371 B1**
(45) Date de publication et mention de la délivrance du brevet: **02.12.2020**
(21) Numéro de dépôt: 18174895.5
(22) Date de dépôt: 11.10.2013
(51) Int. Cl.: C12N 1/20, C12N 1/38

(54) **UTILISATION D'UN COMPOSÉ ANTIOXYDANT POUR LA CULTURE DE BACTÉRIES ANAEROBIES**
VERWENDUNG EINER ANTIOXIDATIONSVERBINDUNG FÜR DIE KULTUR VON ANAEROBEN BAKTERIEN
USE OF AN ANTIOXIDANT COMPOUND FOR THE CULTURE OF ANAEROBIC BACTERIA

(30) Priorité: 22.10.2012 FR 1260020; 06.05.2013 FR 1354134
(43) Date de publication de la demande: 10.10.2018
(62) Demande divisionnaire de: 13786703.2
(73) Titulaire: Fondation Méditerranée Infection, 13005 Marseille (FR)
(72) Inventeur: DRANCOURT, Michel, 13012 Marseille (FR); LA SCOLA, Bernard, 13790 Chateauneuf Le Rouge (FR); RAOULT, Didier, 13008 Marseille (FR)
(74) Mandataire: Cabinet Beau de Loménie

(56) Documents cités:
- I J KLIGLER ET AL: "THE INFLUENCE OF VITAMIN C ON THE GROWTH OF ANAEROBES IN THE PRESENCE OF AIR, WITH SPECIAL REFERENCE TO THE RELATIVE SIGNIFICANCE OF EH AND 02 IN THE GROWTH OF ANAEROBES", JOURNAL OF BACTERIOLOGY, vol. 35, no. 2, 1 février 1938 (1938-02-01), pages 141-156, XP055362096,
- B P EDDY ET AL: "Interactions between ascorbic acid and bacteria", BACTERIOLOGICAL REVIEWS, vol. 17, no. 2, 1 juin 1953 (1953-06-01), pages 93-107, XP055417653, US ISSN: 0005-3678
- WINKLER B S ET AL: "The redox couple between glutathione and ascorbic acid: A chemical and physiological perspective", FREE RADICAL BIOLOGY AND MEDICINE, ELSEVIER INC, US, vol. 17, no. 4, 1 October 1994 (1994-10-01), pages 333-349, XP023522191, ISSN: 0891-5849, DOI: 10.1016/0891-5849(94)90019-1 [retrieved on 1994-10-01]
- D. W. Stainer ET AL: "A Simple Chemically Defined Medium for the Production of Phase I Bordetella pertussis", JOURNAL OF GENERAL MICROBIOLOGY, vol. 63, no. 2, 1 October 1970 (1970-10-01), pages 211-220, XP055674375, GB ISSN: 0022-1287, DOI: 10.1099/00221287-63-2-211

## Description

La présente invention concerne la culture en milieu acellulaire de bactéries dont la croissance est sensible à la tension en oxygène, notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air voire des bactéries anaérobies strictes pour lesquelles l'oxygène est toxique et qui doivent être cultivées en absence totale d'oxygène ou ne tolérant que de faibles concentrations d'oxygène. Il convient en effet de noter que certaines bactéries considérées comme anaérobie strictes peuvent parfois tolérer de faibles concentrations en oxygène.

On distingue donc parmi les bactéries sensibles à l'oxygène :
- les bactéries microaérophiles c'est-à-dire qu'elles ne sont pas capables de cultiver sous une atmosphère comprenant la concentration d'oxygène ambiante qui est de environ 21%, notamment entre 1% et 20%, le plus communément à environ 2-2,5%, et
- les bactéries anaérobies strictes c'est-à-dire qu'elles ne sont pas capables de cultiver en présence d'oxygène ou dans des concentrations inférieures aux concentrations de microaérophilie, notamment strictement inférieure à 1%, le plus communément inférieure à 0.1%, idéalement 0%. Pour cultiver les bactéries anaérobies strictes, il faut soit les cultiver dans des étuves ne comportant pas d'oxygène, soit dans des tubes qui ont été désoxygénés et elles ne poussent alors qu'au fond du tube.

Parmi les bactéries anaérobies strictes, on cite plus particulièrement les bactéries extracellulaires, c'est à dire des bactéries qui qui ne peuvent vivre qu'à l'extérieur de cellules.

Parmi les bactéries cultivables en atmosphère microaérophile, on distingue plus particulièrement, les bactéries intracellulaires, mais aussi des bactéries extracellulaires.

On entend ici par «bactérie intracellulaire», une bactérie qui a la capacité de se multiplier au sein d'une cellule hôte. Les bactéries intracellulaires, ayant la faculté de croître dans certaines conditions dans des milieux acellulaires, sont dénommées "bactéries intracellulaires facultatives".

On entend ici par «bactérie intracellulaire facultative », une bactérie qui a la capacité de se multiplier au sein d'une cellule hôte et en milieu acellulaire.

On entend ici par «bactérie extracellulaire, une bactérie qui n'a pas la capacité de se multiplier au sein d'une cellule hôte et se cultive exclusivement en milieu acellulaire.

On entend ici par «milieu de culture acellulaire», un milieu de culture qui ne comprend pas de cellules entières notamment qui ne comprend pas de cellules hôtes entières au sein desquelles la dite bactérie peut se multiplier, lorsque ladite bactérie est intracellulaire ou intracellulaire facultative.

On comprend que les cellules entières doivent être vivantes pour permettre une multiplication de la bactérie en leur sein.

Plus particulièrement, la présente invention concerne la culture de bactéries anaérobies et la culture de bactéries microaérophiles intracellulaires.

Deux possibilités existent aujourd'hui afin de contrôler la concentration en oxygène dans l'atmosphère utilisée pour l'isolement et la culture des bactéries cultivables en microaérophilie ou en anaérobiose. La première consiste à utiliser des dispositifs de type Gaspak (Oxoid) à base de molécules réductrices qui réagissent et consomment l'oxygène incubé en présence des milieux de culture dans des récipients hermétiques. Un inconvénient de cette approche est qu'elle ne permet pas d'obtenir des concentrations précises en oxygène, ce qui est crucial notamment dans le cas des espèces microaérophiles. La deuxième solution consiste à placer les cultures dans des étuves à concentrations contrôlées en oxygène. Cependant celles-ci sont coûteuses et requièrent une consommation importante en azote ou autre gaz.

Dans l'article Krieg et al. [10], on décrit l'effet de composés oxydants sur la culture de bactéries microaérophiles extracellulaires, notamment Campylobacter jejuni, pour laquelle la cystéine et l'acide ascorbique n'améliorent pas l'aérotolérance (cf. p. 113, 4^{ème} paragraphe :"*cystéine, thioglycolate, mercaptoethanol, thiourea and ascorbic acid do not enhance aerotolerance of C. jejuni on brucella agar and some of these actually inhibit growth...In the case of cysteine...the inhibitory action is likely due to generation of H₂O₂ during autooxidation").*

L'article Omsland et al. [3] concerne le milieu de culture axénique ACCM contenant de la *L. cystéine.* Il est indiqué que le remplacement de la *L. cystéine* par le glutathion ne permet pas de soutenir la croissance de *Coxiella burnetii* (cf. page 4433, colonne de gauche, fin du 1er paragraphe *:"ACCM, containing the alternative antioxydant L.* glutathion *instead of L. cysteine, did not support C. burnetii growth").*

L'article de Podkopaeva et al. [11] concerne la culture de la bactérie microaérophile extracellulaire *Spirillum winogradskii* en présence de thiosulfate.

L'article Eddy et al. (Bacteriological Reviews 17(2):93-107, 1953) décrit un procédé de culture de bactéries anaérobiques en présence d'air, grâce à l'ajout d'acide ascorbique. Des concentrations entre 0.02 et 2 g/L sont envisagées.

Le but de la présente invention est d'améliorer et faciliter les conditions de croissance en culture acellulaire des bactéries dont la croissance est sensible à la tension en oxygène et notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air, lesdites bactéries étant choisies parmi les bactéries anaérobies, y compris les bactéries anaérobies strictes.

On entend ici par «atmosphère microaérophile», de l'air appauvri en oxygène avec une proportion molaire en oxygène inférieure à 10%, de préférence 5%, de préférence encore inférieure à 2,5%. Pour les bactéries anaérobies strictes, la teneur en oxygène doit être proche de 0%, notamment inférieure à 0.1%, comme mentionné ci-dessus, la tolérance à de très faibles quantités d'oxygène étant variable selon les espèces de bactéries anaérobies.

Les inventeurs ont découvert de façon fortuite que l'ajout de certains composés antioxydants dans un milieu de culture acellulaire pouvait permettre de:
- améliorer la croissance des dites bactéries en obtenant plus rapidement des bactéries en concentration suffisante pour être détectables après multiplication et/ou en augmentant la concentration en bactéries après un délai donné de culture, c'est-à-dire par unité de temps, et
- cultiver avec au moins un même niveau de croissance voire à un niveau plus élevé, des bactéries anaérobies strictes en présence de quantité plus élevées d'oxygène qu'en l'absence de composé antioxydant, c'est-à-dire en atmosphère microaérophile, notamment avec des teneurs en oxygène de 2 à 5%, mais aussi des teneurs en oxygène supérieures à 5%, voire en aérobie, c'est-à-dire en présence d'un taux d'oxygène équivalent à la tension en oxygène proche de l'air ambiant soit environ 21%.

La présente invention fournit donc un procédé de culture in vitro de bactéries en milieu de culture acellulaire, de bactéries dont la croissance est sensible à la teneur en oxygène, la croissance optimale desdites bactéries exigeant une atmosphère d'incubation à teneur en oxygène relativement réduite, voire nulle, par rapport à la teneur en oxygène de l'air, lesdites bactéries étant choisies parmi les bactéries anaérobies caractérisé en ce que l'on ajoute dans le dit milieu de culture acellulaire de l'acide ascorbique et le glutathion, chacun à une concentration de 500 µg/ml et l'on cultive la dite bactérie dans ledit milieu de culture en présence d'oxygène.

Plus particulièrement, les inventeurs ont découvert que l'ajout d'un composé antioxydant confère un effet tampon au regard de la teneur en oxygène pour les bactéries, notamment les bactéries anaérobies strictes et les bactéries intracellulaires car cet ajout permet de tolérer une croissance en présence de teneur en oxygène relativement plus élevée, notamment pour les bactéries anaérobies strictes. Et, cet ajout permet aussi pour certaines bactéries cultivables à des tensions d'oxygènes plus élevées en l'absence de composé antioxydant, d'améliorer leur croissance à des tensions en oxygène diminuées en présence de composé antioxydant.

Les inventeurs formulent l'hypothèse non encore totalement élucidée et démontrée que l'effet du composé antioxydant pourrait provenir d'une réaction avec les radicaux libres oxygénés toxiques ayant un effet inhibiteur de la toxicité des dits radicaux à l'encontre de la croissance de la dite bactérie. Ces radicaux résultant de l'action de l'oxygène sur des substances issues des bactéries et/ou milieu de culture, seraient responsables des difficultés de culture et mauvaise tolérance de la croissance des dites bactéries en présence de teneurs élevées d'oxygène.

Plus particulièrement, le dit milieu acellulaire est un milieu autre qu'un milieu constitué essentiellement à partir d'un lysat ou broyat cellulaire d'un type de cellules eucaryotes donné, notamment autre qu'un filtrat de dit broyat ou lysat, de préférence un milieu autre qu'un milieu acellulaire de lysat ou broyat cellulaire d'un type de cellules eucaryotes donné au sein desquelles la dite bactérie peut être cultivée, notamment autre qu'un filtrat de dit broyat ou lysat..

Plus particulièrement, le dit milieu acellulaire est choisi parmi un milieu axénique constitué de substances chimiques ou biologiques défini qualitativement et quantitativement, et un milieu comprenant un extrait de broyat ou lysat de tissu pluricellulaire.

Plus particulièrement encore, le dit milieu acellulaire est un milieu axénique constitué de substances chimiques ou biologiques défini qualitativement et quantitativement, lorsque la dite bactérie est une bactérie intracellulaire facultative, et le dit milieu acellulaire est constitué à partir d'un broyat ou lysat de tissu pluricellulaire, notamment un filtrat de dit broyat ou lysat, notamment de tissu sanguin ou tissu de cœur et/ou poumon, lorsque ladite bactérie est une bactérie extracellulaire.

De tels milieux de culture acellulaires qu'ils soient liquides, solides ou biphasiques sont bien connus de l'homme de l'art.

Plus particulièrement, on cultive la dite bactérie dans une dite atmosphère d'incubation comprenant une proportion molaire en oxygène supérieure à la tension maximale tolérée en l'absence de composé antioxydant pour un même niveau de croissance dans une même durée de culture.

En pratique, plus particulièrement encore, on cultive la dite bactérie dans une dite atmosphère d'incubation comprenant une proportion molaire en oxygène inférieure ou égale à 20%.

Avantageusement toutefois, on cultive lesdites bactéries selon la présente invention dans une atmosphère comprenant une teneur en oxygène supérieure à 5%, notamment dans de l'air contenant 5% de CO₂ (soit une teneur en oxygène inférieure à 16%), voire dans une atmosphère aérobie.

Les inventeurs ont en effet testé différentes molécules présentant une activité antioxydante et ont découvert que certains composés anti-oxydants dans certaines concentrations présentent un effet supérieur sur la croissance des dites bactéries.

Plus particulièrement, le dit milieu de culture comprend de l'acide ascorbique et le glutathion (y-L-Glutamyl-L-cysteinylglycine). L'acide ascorbique et le glutathion sont préférés car ils sont capables à des doses précises de permettre la culture à un niveau d'oxygène plus élevé.

Plus particulièrement, le dit milieu de culture comprend comme composé antioxydant additionnel de l'hydrosulfure de sodium

Plus particulièrement, la dite bactérie est une bactérie anaérobie cultivable dans un dit milieu de culture en l'absence de dit composé oxydant sous une atmosphère d'incubation comprenant une proportion molaire en oxygène inférieure à la proportion molaire d'oxygène dans l'air, de préférence inférieure à 20%, et on cultive la dite bactérie en présence de dit composé oxydant dans le dit milieu de culture sous une atmosphère d'incubation comprenant une teneur en oxygène inférieure ou égale à la proportion d'oxygène dans l'air, de préférence inférieure à 20%, de préférence encore supérieure à 5%.

Plus particulièrement, la dite bactérie est une bactérie anaérobie extracellulaire cultivable en atmosphère anaérobie en l'absence de dit composé antioxydant, et l'on obtient une croissance de la dite bactérie en présence d'oxygène avec une proportion molaire inférieure ou égale à la proportion d'oxygène dans l'air.

Parmi les bactéries extracellulaires anaérobies, on cite plus particulièrement les bactéries appartenant aux genres *Peptostreptococcus, Finegoldia, Anaerococcus, Peptoniphilus, Veillonella, Propionibacterium, Lactobacillus, Actinomyces, Clostridium, Bacteroides, Porphyromonas, Prevotella, Firmicutes, Solobacterium, Atopobium, Ruminococcus et Fusobacterium, de préférence les espèces choisies parmi Finegoldia magna, Bacteroides massiliensis, Fusobacterium necrophorum, Finegoldia magna, Prevotella nigrescens, Solobacterium morei, Atopobium vaginae et Ruminococcus gnavus.*

Selon ce premier mode de réalisation plus particulier, on cultive une bactérie extracellulaire anaérobie dans un dit milieu acellulaire en présence d'une proportion molaire d'oxygène inférieure ou égale à celle de l'air et en présence d'un mélange d'acide ascorbique et de gluthation à des concentrations respectives de 500µg/ml chacun.

Plus particulièrement, le dit milieu de culture de bactéries anaérobies peut se trouver sous forme liquide ou de préférence, solide ou semi-solide, notamment gélosé ou semi gélosé, gélosé ou semi gélosé.

Plus particulièrement, ledit milieu de culture est un milieu acellulaire conventionnel de bactérie anaérobie, de préférence un milieu comprenant des composant choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

Plus particulièrement encore, la bactérie est une bactérie anaérobie cultivée en tube dans un milieu conventionnel de culture de bactéries anaérobies tel que les milieux dits bouillon de type cœur-cervelle, milieux Columbia à 5% de sang de mouton ou milieu Schaedler tels que décrits ci-après. D'autres milieux conventionnels appropriés sont les milieux Brucella ou Wilkins-Chagren. Ces milieux sont utilisables avec agar (solide ou semi-solide) ou sans agar (liquide).

Plus particulièrement, lesdites bactéries sont des bactéries anaérobies strictes du tube digestif choisies parmi *Bacteroides massiliensis, Fusobacterium necrophorum, Finegoldia magna, Prevotella nigrescens, Solobacterium morei, Atopobium vaginae, Ruminococcus gnavus.*

Il s'agit de bactéries anaérobies strictes du tube digestif, qui normalement ne se cultivent que dans des conditions strictement anaérobies. Ceci se traduit quand on inocule une dite bactérie dans un tube désoxygéné par le fait qu'un voile de culture n'apparait que dans la partie basse du tube, tandis que la partie haute reste vierge de culture. Si on ajoute, un composé antioxydant selon la présente invention, la culture se fait pratiquement jusqu'à la surface, voire complètement jusqu'à la surface à la concentration de 500µg/ml et encore mieux à 1 mg/l, ou avec un mélange d'acide ascorbique à 500µg/ml et de gluthation à 500µg/ml montrant que l'acide ascorbique à une certaine concentration permet la culture des bactéries anaérobies.

Plus particulièrement encore, le dit milieu de culture axénique est le milieu dénommé ACCM [3] comprenant les composant suivants aux concentrations suivantes: tampon citrate (acide citrique: 2,5 g/ml; citrate de sodium tribasique: 4,74 mg/ml); du phosphate de potassium (0,5 mg/ml), du chlorure de magnésium (0,2 mg/ml), du chlorure de calcium (0,013 mg/ml), du sulfate de fer (2 µg/ml), de la L-cystéine (0,26 mg/ml), de la néopeptone (0,1 mg/ml), des casamino acides (2,5 mg/ml), de la méthyl béta cyclodextrine (1 mg/ml). Ce milieu de culture est un milieu chimiquement défini décrit dans WO 2010/096640 dénommé ACCM (ACCM=acidified citrate cystéine medium) amélioré en AACM-2 adapté aux bactéries pathogènes donnant comme exemple C. *burnetii.* Ce milieu a permis l'obtention de colonies (0,01 mm de diamètre) en 6 jours.

Selon la présente invention, en présence de composé antioxydant dans un dit milieu axénique, sous une atmosphère à proportion molaire en oxygène inférieure à 20%, notamment inférieure à 16% (de l'air enrichi à 5% de CO₂), on peut multiplier la concentration de bactérie *Coxiella burnetii* d'au moins un facteur 10 (1 log) en pas plus de 9 jours.

Les milieux solides sont fabriqués à base de gélose ou d'agar. Les milieux contenant de l'œuf entier sont d'utilisation très courante et le milieu le plus utilisé est le milieu de Lowenstein Jensen. Une catégorie de milieux solides sont les milieux à l'agar en particulier le milieu Middlebrook 7H10 et le milieu 7H11 (milieu 7H10 plus 0,1% de caséine hydrolysée).

Plus particulièrement encore, ledit milieu de culture est un milieu de culture solide type Middlebrook de référence 7H10, additionné de produit gélifiant choisi de préférence parmi les géloses et agar, de préférence dans une proportion pondérale de 0.5 à 5%, de préférence encore de 1 à 2%.

En pratique, le milieu de culture se présente initialement sous forme d'un lyophilisat desdits composants listés ci-dessus, destiné à être dilué dans de l'eau distillée pour former le milieu de culture selon l'invention.

D'autres caractéristiques et avantages de l'invention sont présentés dans la description détaillée et les exemples qui suivent.

### Exemple 1 : Effet de l'acide ascorbique, du gluthation et du mélange acide ascorbique+glutathion sur la croissance axénique des bactéries anaérobies du genre Bacteroides.

On a utilisé *Bacteroides massiliensis* (CCUG48901) [7] isolée pour la première fois en 2005 dans des hémocultures et depuis dans des prélèvements de selles.

La croissance de souches de la dite bactérie strictement anaérobie c'est à dire cultivant habituellement en absence totale d'oxygène, a été testée en présence d'une faible tension en oxygène.

Un inoculum de 10⁷ bactéries /mL a été inoculé sur toute la hauteur d'un tube d'un milieu de culture du type bouillon cœur-cervelle contenant 5 mL d'infusion de cœur-cervelle («Brain Heart Infusion») avec 5% d'agar (Becton Dickinson, Le Pont-de-Claix, France), en tube à vis enrichi avec 100 µg/ml d'acide ascorbique.

Le milieu cœur cervelle présentait la composition suivante pour 1 litre:

| | |
|---|---|
| -Infusion solide de cervelle | 12.5 g |
| -Infusion solide de cœur de bœuf | 5 g |
| -Proteose peptone | 10 g |
| -Glucose | 2 g |
| -NaCl | 5 g |
| -Phosphate disodique | 2.5 g |

Les tubes ont été incubés à 37°C dans une étuve anaérobie stricte durant 48 heures.

Dans ces conditions, il a été observé une croissance habituelle de B. massiliensis depuis le fond du tube jusqu'à 1 cm en dessous de la surface du bouillon en absence d'acide ascorbique témoignant du caractère anaérobie de cette bactérie, et une croissance jusqu'à 2 mm de la surface en présence de 100 µg/mL (5,6 X 10⁻⁴ M) d'acide ascorbique indiquant une croissance en présence d'une tension d'oxygène plus grande qu'en l'absence d'acide ascorbique.

### Exemple 2 : Effet de l'acide ascorbique sur la croissance axénique d'autres bactéries anaérobies des genres Fusobacterium, Finegoldia, Prevotella, Solobactreium, Atopobium et Ruminococcus.

Dans cet exemple, la croissance de souches de bactéries strictement anaérobies c'est à dire cultivant en absence totale d'oxygène, a été testée en présence de concentrations variables en oxygène. Ces bactéries sont *Fusobacterium necrophorum, Finegoldia magna, Prevotella nigrescens, Solobactreium morei, Atopobium vaginae, Ruminococcus gnavus,* toutes isolée en situation pathogène chez l'homme [9].

La procédure de culture est réalisée sur des tubes de milieu de culture du type Schaedler avec 0,2% d'agar (Biomerieux, Marcy l'étoile, France).

Le milieu Schaedler présentait la composition suivante pour 1 litre:

| | |
|---|---|
| -Digestat enzymatique de caséine | 5.6 g |
| -Digestat enzymatique de tourteau de soja | 1 g |
| -Digestat enzymatique de tissus animal | 5 g |
| -Extrait de levure | 5 g |
| -NaCl | 1.7 g |
| -Phosphate de potassium | 0.82 g |
| -Dextrose | 5.82 g |
| -Tris (hydroxymethyl) Aminomethane | 3 g |
| -Hemine | 0.01 g |
| -L-cysteine | 0.4g |
| -Agar (milieu semi-solide) | 0.2% |

Pour chaque bactérie on inocule 2 tubes, un tube régénéré sans acide ascorbique et un tube régénéré dans lequel on ajoute 500µg/ml ou 1mg/ml d'acide ascorbique. Pour régénérer le tube, on le place au bain marie à 100°C jusqu'à ce que toutes les bulles de gaz visibles dans le milieu aient disparu. Ensuite, pour le tube avec acide ascorbique, on attend le refroidissement du tube à 50°C (schématiquement jusqu'à pouvoir le tenir dans la main sans se brûler) et on ajoute la suspension d'acide ascorbique. On homogénéise ensuite par retournement (3-4 pour assurer un bon mélange). Pour chaque bactérie un inoculum de 10⁷ bactéries /ml a été inoculé sur toute la hauteur des tubes Schaedler 0,2%, un normal et un complémenté en acide ascorbique. Les tubes ont été incubés à 37°C dans une étuve anaérobie stricte durant 24-48 heures.

Dans ces conditions, il a été observé une croissance habituelle des bactéries depuis le fond du tube jusqu'à 1,5 cm en dessous de la surface du milieu en absence d'acide ascorbique témoignant du caractère anaérobie de cette bactérie, et une croissance jusqu'à la surface en présence de 500 µg/ml (28 X 10⁻⁴ M) d'acide ascorbique indiquant une croissance en présence d'une tension d'oxygène plus grande qu'en l'absence d'acide ascorbique.

Des tests en tous points identiques ont été réalisés soit avec du gluthation à 500µg/l soit avec un mélange gluthation à 500µg/l + acide ascorbique à 500µg/l. Avec le gluthation seul, la croissance est identique à ce qui est observé avec l'acide ascorbique. Avec le mélange gluthation+acide ascorbique, la croissance observée sous la surface est encore plus intense que ce qui est observé avec chaque composé individuellement.

Enfin, afin de valider définitivement la capacité de ces composés à autoriser la croissance de bactéries anaérobies strictes en présence d'oxygène, on a préparé des milieux solides constitués de milieu Columbia avec 5% de sang de sang de mouton dans lesquels ont été ajouté du gluthation à 500µg/l, ou un mélange gluthation à 500µg/l + acide ascorbique à 500µg/l ou de l'acide ascorbique à 1mg/l. Ces géloses inoculées de bactéries anaérobies ont été incubées soit en air ambiant soit en air ambiant enrichi de 5% de CO2. Bien que fine, une pousse a été observée avec les seuls milieux complémentés avec les antioxydants, la meilleure pousse ayant été obtenue avec de l'acide ascorbique à 1mg/l.

Ces tests ont été réalisés un milieu Columbia 5% de sang de mouton présentant la composition suivante pour 1 litre:

| | |
|---|---|
| -Digestat enzymatique de caséine | 5 g |
| -Digestat enzymatique de tissus animal | 8 g |
| -Peptone enrichie de levure | 10 g |
| -Amidon de maïs | 1 g |
| -NaCl | 5 g |
| -Agar (si milieu gélosé) | 14 g |
| -Sang de mouton | 5% |

### Références bibliographiques

[1] Joshi P, Singh M, Bhargava A, Singh M, Mehrotra R. Autofluorescence-an important ancillary technique for the detection of Mycobacterium tuberculosis: Revisited. Diagn Cytopathol. 2012 Feb 20. doi: 10.1002/dc.21860. [Epub ahead of print].
[2] Patiño S, Alamo L, Cimino M, Casart Y, Bartoli F, Garcia MJ, Salazar L. Autofluorescence of mycobacteria as a tool for detection of Mycobacterium tuberculosis. J Clin Microbiol. 2008;46:3296-302.
[3] Omsland A, Cockrell DC, Howe D, et al. Host cell-free growth of the Q fever bacterium Coxiella burnetii. Proc. Natl. Acad. Sci. U.S.A. 2009; 106:4430-4434.
[4] Gimenez DF. STAINING RICKETTSIAE IN YOLK-SAC CULTURES72. Stain Technol 1964 May;39:135-40.
[5] Angelakis E, Richet H, Rolain J-M, La Scola B, Raoult D. Comparison of real-time quantitative PCR and culture for the diagnosis of emerging Rickettsioses. PLoS Negl Trop Dis 2012; 6:e1540.
[6] El Khéchine A, Couderc C, Flaudrops C, Raoult D, Drancourt M. Matrix-assisted laser desorption/ionization time-of-flight mass spectrometry identification of mycobacteria in routine clinical practice. PLoS One. 2011;6(9):e24720).
[7] Fenner L, Roux V, Mallet MN, Raoult D. Bacteroides massiliensis sp. nov., isolated from blood culture of a newborn. Int J Syst Evol Microbiol. 2005 May;55:1335-7.
[8] Fenollar F, Fournier PE, Raoult D. Molecular detection of Coxiella burnetii in the sera of patients with Q fever endocarditis or vascular infection. J Clin Microbiol 2004; 42:4919-24).
[9] La Scola B, Fournier PE, Raoult D. Burden of emerging anaerobes in the MALDI-TOF and 16S rRNA gene sequencing era. Anaerobe 2011 ; 17:106-112
[10] N R Krieg et al. Microaerophily and oxygen toxicity. Annual review of microbiology, vol. 40, n°1, 1986, pages 107-130
[11] D. A. Podkopaeva et al. Oxidative stress and antioxidant cell protection systems in the microaerophilic bacterium Spirillum winogradskii. Microbiology, vol. 72, n°5, 2003, pages 534-541

## Revendications

1. Procédé de culture *in vitro* en milieu de culture acellulaire, de bactérie dont la croissance est sensible à la teneur en oxygène, la croissance optimale de la dite bactérie exigeant une atmosphère d'incubation à teneur en oxygène relativement réduite par rapport à la teneur en oxygène de l'air, ladite bactérie étant choisie parmi les bactéries anaérobies, **caractérisé en ce que** l'on ajoute dans le dit milieu de culture un mélange de composés antioxydants comprenant au moins l'acide ascorbique et le glutathion, chacun à une concentration de 500 µg/ml, et l'on cultive la dite bactérie dans ledit milieu de culture en présence d'oxygène.

2. Procédé selon la revendication 1, **caractérisé en ce que** le dit milieu acellulaire est choisi parmi :
- un milieu axénique constitué de substances chimiques ou biologiques défini qualitativement et quantitativement, et
- un milieu comprenant un extrait de broyat ou lysat de tissu pluricellulaire.

3. Procédé selon l'une des revendications 1 ou 2 , **caractérisé en ce que** l'on cultive la dite bactérie dans une dite atmosphère d'incubation comprenant une proportion molaire en oxygène supérieure à la tension maximale tolérée en l'absence de composé antioxydant pour un même niveau de croissance dans une même durée de culture.

4. Procédé selon l'une des revendications 1 à 3, **caractérisé en ce que** la dite bactérie est une bactérie anaérobie extracellulaire cultivable en atmosphère anaérobie en l'absence de dit composé antioxydant, et l'on obtient une croissance de la dite bactérie en présence d'oxygène avec une proportion molaire inférieure ou égale à la proportion d'oxygène dans l'air.

5. Procédé selon la revendication 4 **caractérisé en ce qu'**on obtient une croissance de la dite bactérie en air ambiant enrichi de 5% de CO₂.

6. Procédé selon l'une des revendications 1 à 5, **caractérisé en ce que** les dites bactéries anaérobies sont des bactéries anaérobies strictes du tube digestif.

7. Procédé selon l'une des revendications 1 à 6, **caractérisé en ce que** la dite bactérie extracellulaire anaérobie est choisie parmi les bactéries appartenant aux genres *Peptostreptococcus, Finegoldia, Anaerococcus, Peptoniphilus, Veillonella, Propionibacterium, Lactobacillus, Actinomyces, Clostridium, Bacteroides, Firmicutes, Porphyromonas, Prevotella, Fusobacterium, Atopobium, Ruminococcus et Solobacterium.*

8. Procédé selon la revendication 7 **caractérisé en ce que** la dite bactérie est choisie parmi *Bacteroides massiliensis, Fusobacterium necrophorum, Finegoldia magna, Prevotella nigrescens, Solobacterium morei, Atopobium vaginae, et Ruminococcus gnavus.*

9. Procédé selon l'une des revendications 1 à 8, **caractérisé en ce que** l'on cultive une bactérie extracellulaire anaérobie dans un dit milieu acellulaire, en présence d'une proportion molaire d'oxygène inférieure ou égale à celle de l'air, en présence de dit composé antioxydant comprenant un mélange de glutathion et d'acide ascorbique.

10. Procédé selon l'une des revendications 1 à 9, **caractérisé en ce que** ledit milieu de culture est un milieu acellulaire conventionnel de bactérie anaérobie, comprenant des composants choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

11. Procédé selon l'une des revendications 1 à 10, **caractérisé en ce que** ledit milieu de culture est un milieu liquide.

12. Procédé selon l'une des revendications 1 à 10, **caractérisé en ce que** le dit milieu de culture est un milieu solide ou semi solide.

13. Procédé selon la revendication 12 **caractérisé en ce que** ledit milieu est un milieu solide gélosé contenant de préférence au moins 14 g/L d'agar.

## Patentansprüche

1. Verfahren zur *In-vitro*-Kultur in einem azellulären Kulturmedium eines Bakteriums, dessen Wachstum gegenüber dem Sauerstoffgehalt empfindlich ist, wobei das optimale Wachstum des Bakteriums eine Inkubationsatmosphäre mit einem im Vergleich zum Sauerstoffgehalt der Luft relativ niedrigen Sauerstoffgehalt erfordert, wobei das Bakterium ausgewählt ist aus anaeroben Bakterien, **dadurch gekennzeichnet, dass** eine Mischung von antioxidativen Verbindungen, die mindestens Ascorbinsäure und Glutathion umfassen, dem Kulturmedium jeweils in einer Konzentration von 500 µg/ml zugesetzt wird und das Bakterium in dem Kulturmedium in Gegenwart von Sauerstoff kultiviert wird.

2. Verfahren nach Anspruch 1, **dadurch gekennzeichnet, dass** das azelluläre Medium ausgewählt ist aus:
- einem axenischen Medium, das aus chemischen oder biologischen Substanzen besteht, die qualitativ und quantitativ definiert sind, und
- einem Medium, das ein Extrakt aus zerkleinertem oder lysiertem multizellulärem Gewebe umfasst.

3. Verfahren nach einem der Ansprüche 1 oder 2, **dadurch gekennzeichnet, dass** das Bakterium in einer Inkubationsatmosphäre kultiviert wird, die einen molaren Anteil an Sauerstoff umfasst, der höher als der maximale Druck ist, der in Abwesenheit einer antioxidativen Verbindung für das gleiche Wachstumsniveau in der gleichen Kulturzeit toleriert wird.

4. Verfahren nach einem der Ansprüche 1 bis 3, **dadurch gekennzeichnet, dass** es sich bei dem Bakterium um ein extrazelluläres anaerobes Bakterium handelt, das in einer anaeroben Atmosphäre in Abwesenheit der antioxidativen Verbindung kultiviert werden kann, und ein Wachstum des Bakteriums in Gegenwart von Sauerstoff mit einem molaren Anteil, der kleiner oder gleich dem Anteil des Sauerstoffs in Luft ist, erhalten wird.

5. Verfahren nach Anspruch 4, **dadurch gekennzeichnet, dass** ein Wachstum des Bakteriums in mit 5 % CO₂ angereicherter Umgebungsluft erhalten wird.

6. Verfahren nach einem der Ansprüche 1 bis 5, **dadurch gekennzeichnet, dass** es sich bei den anaeroben Bakterien um strikt anaerobe Bakterien des Verdauungstraktes handelt.

7. Verfahren nach einem der Ansprüche 1 bis 6, **dadurch gekennzeichnet, dass** das anaerobe extrazelluläre Bakterium aus Bakterien ausgewählt wird, die zu den Gattungen *Peptostreptococcus, Finegoldia, Anaerococcus, Peptoniphilus, Veillonella, Propionibacterium, Lactobacillus, Actinomyces, Clostridium, Bacteroides, Firmicutes, Porphyromonas, Prevotella, Fusobacterium, Atopobium, Ruminococcus und Solobacterium gehören.*

8. Verfahren nach Anspruch 7, **dadurch gekennzeichnet, dass** das Bakterium ausgewählt ist aus *Bacteroides massiliensis, Fusobacterium necrophorum, Finegoldia magna, Prevotella nigrescens, Solobacterium morei, Atopobium vaginae und Ruminococcus gnavus.*

9. Verfahren nach einem der Ansprüche 1 bis 8, **dadurch gekennzeichnet, dass** ein anaerobes extrazelluläres Bakterium in einem besagten azellulären Medium in Gegenwart eines molaren Anteils an Sauerstoff, der kleiner oder gleich dem der Luft ist, in Gegenwart der antioxidativen Verbindung, die eine Mischung aus Glutathion und Ascorbinsäure umfasst, kultiviert wird.

10. Verfahren nach einem der Ansprüche 1 bis 9, **dadurch gekennzeichnet, dass** es sich bei dem Kulturmedium um ein herkömmliches azelluläres Medium aus anaeroben Bakterien handelt, das Komponenten umfasst, die ausgewählt sind aus einem Extrakt aus zerkleinertem oder lysiertem multizellulärem Gewebe, einem enzymatischen Gärrest, insbesondere einem enzymatischen Gärrest aus Casein, Sojabohnen und/oder tierischem Gewebe, einem Pepton, einem Hefeextrakt, einem Zucker wie Dextrose oder Glucose, einem NaCl- und/oder Na₂PO₄-Salz.

11. Verfahren nach einem der Ansprüche 1 bis 10, **dadurch gekennzeichnet, dass** es sich bei dem Medium um ein flüssiges Medium handelt.

12. Verfahren nach einem der Ansprüche 1 bis 10, **dadurch gekennzeichnet, dass** es sich bei dem Medium um ein festes oder halbfestes Medium handelt.

13. Verfahren nach Anspruch 12, **dadurch gekennzeichnet, dass** es sich bei dem Medium um ein festes Gelmedium handelt, das vorzugsweise mindestens 14 g/l Agar enthält.

## Claims

1. A process for the *in vitro* culture, in cell-free culture medium, of a bacterium whose growth is sensitive to the oxygen content, the optimal growth of said bacterium requiring an incubation atmosphere with a relatively low oxygen content compared with the oxygen content of the air, said bacterium being selected from anaerobic bacteria, **characterized in that** a mixture of antioxidant compounds comprising at least ascorbic acid and glutathione, each at a concentration of 500 µg/ml, is added to said culture medium, and said bacterium is cultured in said culture medium in the presence of oxygen.

2. The process as claimed in claim 1, **characterized in that** said cell-free medium is selected from:
- an axenic medium consisting of qualitatively and quantitatively defined chemical or biological substances, and
- a medium comprising an extract of ground or lysed multicellular tissue.

3. The process as claimed in one of claims 1 or 2, **characterized in that** said bacterium is cultured in a said incubation atmosphere comprising a molar proportion of oxygen greater than the maximum tension tolerated in the absence of antioxidant compound for the same level of growth in the same culture period.

4. The process as claimed in one of claims 1 to 3, **characterized in that** said bacterium is an extracellular anaerobic bacterium that can be cultured in an anaerobic atmosphere in the absence of said antioxidant compound, and growth of said bacterium is obtained in the presence of oxygen with a molar proportion less than or equal to the proportion of oxygen in the air.

5. The process as claimed in claim 4, **characterized in that** a growth of said bacterium is obtained in ambient air enriched with 5% CO₂.

6. The process as claimed in one of claims 1 to 5, **characterized in that** said anaerobic bacteria are obligate anaerobic bacteria of the digestive tract.

7. The process as claimed in one of claims 1 to 6, **characterized in that** said extracellular anaerobic bacterium is selected from bacteria belonging to the genera *Peptostreptococcus, Finegoldia, Anaerococcus, Peptoniphilus, Veillonella, Propionibacterium, Lactobacillus, Actinomyces, Clostridium, Bacteroides, Firmicutes, Porphyromonas, Prevotella, Fusobacterium, Atopobium, Ruminococcus* and *Solobacterium.*

8. The process as claimed in claim 7, **characterized in that** said bacterium is selected from *Bacteroides massiliensis, Fusobacterium necrophorum, Finegoldia magna, Prevotella nigrescens, Solobacterium morei, Atopobium vaginae,* and *Ruminococcus gnavus.*

9. The process as claimed in one of claims 1 to 8, **characterized in that** an extracellular anaerobic bacterium is cultured in a said cell-free medium, in the presence of a molar proportion of oxygen less than or equal to that of the air, in the presence of said antioxidant compound comprising a mixture of glutathione and ascorbic acid.

10. The process as claimed in one of claims 1 to 9, **characterized in that** said culture medium is a conventional cell-free medium for anaerobic bacterium, comprising components selected from an extract of ground or lysed multicellular tissue, an enzyme digestate, in particular an enzyme digestate of casein, of soybean and/or of animal tissue, a peptone, a yeast extract, a sugar such as dextrose or glucose, a NaCl and/or Na₂PO₄ salt.

11. The process as claimed in one of claims 1 to 10, **characterized in that** said culture medium is a liquid medium.

12. The process as claimed in one of claims 1 to 10, **characterized in that** said culture medium is a solid or semi-solid medium.

13. The process as claimed in claim 12, **characterized in that** said medium is a solid agar medium preferably containing at least 14 g/L of agar.
